Europäisches Patentamt

European Patent Office

Office européen des brevets

(19)

(11) Publication number: **0 080 806**
**B1**

## EUROPEAN PATENT SPECIFICATION

(12)

(45) Date of publication of patent specification: **19.07.89**

(21) Application number: **82305632.0**

(22) Date of filing: **22.10.82**

(51) Int. Cl.[4]: **C 12 N 15/00,** A 61 K 39/02,
A 61 K 39/00, C 12 N 1/00,
C 12 N 9/10 // C12R1/19

(54) **Live vaccine and its method of production.**

(30) Priority: **22.10.81 US 314030**

(43) Date of publication of application:
**08.06.83 Bulletin 83/23**

(45) Publication of the grant of the patent:
**19.07.89 Bulletin 89/29**

(84) Designated Contracting States:
**AT BE CH DE FR GB IT LI LU NL SE**

(56) References cited:
**EP-A-0 001 930**
**EP-A-0 027 662**
**EP-A-0 048 455**
**EP-A-0 060 129**
**EP-A-0 068 693**
**EP-A-0 068 719**
**DE-A-2 530 275**
**FR-A-2 282 908**
**FR-A-2 442 271**
**GB-A-2 033 223**
**US-A-4 133 875**
**US-A-4 237 224**

**The file contains technical information
submitted after the application was filed and
not included in this specification**

(73) Proprietor: **Research Corporation
405 Lexington Avenue
New York, N.Y. 10174 (US)**

(72) Inventor: **Curtiss III, Roy
2901 Smyer Road
Birmingham Alabama 35216 (US)**

(74) Representative: **Sheard, Andrew Gregory et al
Kilburn & Strode 30, John Street
London WC1N 2DD (GB)**

(56) References cited:
**NATURE, vol. 242, 20th April 1973, pages
531-532;**
**CHEMICAL ABSTRACTS, vol. 94, 1981, page
374, no. 170896e, Columbus Ohio (USA); J.B.
HANSEN et al.: "Characterization of the
streptococcus mutans plasmid pVA318 cloned
into escherichia coli"**
**CHEMICAL ABSTRACTS, vol. 96, 1982, page
116, no. 46808e, Columbus Ohio (USA);**

EP 0 080 806 B1

Courier Press, Leamington Spa, England.

(56) References cited:

S.B.FORMAL et al.: "Construction of a potential bivalent vaccine strain: introduction of shigella sonnei form I antigen genes into the galE salmonella typhi Ty21 a typhoid vaccine strain" NATURE, vol. 238, 28th July 1972, page 219;

S.J.CHALLACOMBE et al.: "Serum and salivary antibodies to glucosyltransferase in dental caries in man"

NATURE, vol. 289, 19th February 1981, pages 696-698; R.P. SILVER et al.: "Molecular cloning of the KI capsular polysaccharide genes of E. coli"

CHEMICAL ABSTRACTS, vol. 88, 1978, page 240, no. 117440b, Columbus Ohio (USA); Y.KEIJI: "Application of plasmids, focusing on the genus pseudomonas. A review"

CHEMICAL ABSTRACTS, vol. 93, 1980, page 460, no. 65528t, Columbus Ohio (USA); M.LOVETT et al.: "Plasmids of the genus chlamydia"

CHEMICAL ABSTRACTS, vol. 91, 1979, page 347, no. 171499c, Columbus Ohio (USA); M.C. ROBERTS: "Plasmids and conjugation in neisseria gonorrhosa"

CHEMICAL ABSTRACTS, vol. 91, 1979, page 298, no. 35502w, Columbus Ohio (USA); E.S.CREEGER et al.: "Cloning of gones for bacterial glycosytransferases. I. Selection of hybrid plasmids carrying genes for two glucosyitransferases"

J.M.RUTTER et al.: "Protection against enteric disease caused by Escherichia coli - a model for vaccination with a virulence determinant" Nature, vol. 280, 30.8.79, pp. 815-819 Nature, vol. 286, 28.8.80, pp. 893-895 Proc.Natl.Acad.Sci., USA, vol. 77 (1980), pp. 5507-5511

J.Bacteriol., vol. 128 (1), 1976, pp. 463-472 Infection and Immunity, vol. 29 (3), 1980, pp. 1125-1133 Nature, vol. 288, 4.Dec.1980, pp. 499-501 J.Clin.Invest., vol. 71 (1983), pp. 1-8

# EP 0 080 806 B1

**Description**

The invention described herein was made in the course of work supported by a grant from the Department of Health, Education, and Welfare, Public Health Service.

The present invention relates to vaccines and more particularly to antigenic gene products obtained from microbes containing one or more recombinant genes from a pathogenic organism as a vaccine against the pathogenic organism.

Bacterial infectious diseases are ubiquitous, although improved public health and the availability of antibiotics have decreased the incidence and minimized the consequences of infectious bacterial diseases in the world. In many underdeveloped countries, however, bacterial diseases are still rampant. Even in medically advanced countries, bacterial transposons that carry drug-resistance genes and numerous methods for dissemination of drug-resistance plasmids have resulted in drug resistance that has compromised the effectiveness of conventional methods of infectious disease control designed to counteract the effects of infection after infection has occurred. Thus, in the last several years, there has been increased emphasis on the development of vaccines that might prevent, if not eliminate, some infectious diseases.

Since the intact surface of a healthy epidermis is rarely, if ever, penetrated by bacteria, the point of initial infection is most often one of the mucous membranes. These include the conjunctiva and the oral, respiratory, gastrointestinal, and genitourinary surfaces. The mucous secretions of these membranes contain antibodies against invading pathogens and thus act as a first line of defense against invasive bacteria. Enhancing the immune response of the secretory system is thus a desirable goal in inducing immunity against pathogens.

Previously used vaccines against bacterial diseases have generally comprised (I) specific components purified from the aetiologic agents, (II) the whole killed aetiologic agent, or (II) an avirulent derivative of the aetiologic agent as a live vaccine. Numerous vaccines of these three types exist, of which the following are selected examples:

U.S. 4,250,262, discloses methods for recovering the enzyme glucosyltransferase from *Streptococcus mutans* and the use of this purified enzyme in local immunization against dental caries, a Type I vaccine. Details for culturing the bacteria, purifying the enzyme, and using the enzyme to stimulate IgA antibody in saliva are presented for serotype a, c or g of *S. mutans*. Other examples of vaccines from purified specific components of bacteria are found in U.S. Patents 4,203,971 and 4,239,749, which disclose a vaccine useful against infection by *Neisseria gonorrhoeae* which consists of a glycoprotein from the outer coat material of gonococci. Injection of the glycoprotein stimulates a bactericidal antibody.

The use of dead *S. mutans* cells to immunize against tooth decay via administering in the mouth, which is disclosed in U.S. Patent 3,931,398, is an example of a Type II vaccine. The inventors recognized that immunoglobulin A (IgA) antibodies were the antibodies being produced and that they resulted in a decrease in plaque formation.

A live bacterial vaccine (Type III) which contains selected strains of *Escherichia coli* bacteria is disclosed in U.S. Patent 3,975,517. The bacteria were treated with dilute formalin to attenuate or partially inactivate them before injection into the mammary gland of a sow. Antibody thereby produced was later found in the milk and protected newborn swine against *E. coli* infections. The formalin treatment that caused the *E. coli* inactivation was only a temporary attenuation of the bacteria and care had to be taken to prevent bacterial recovery before injection. Such recovery would have resulted in serious infection rather than protection.

Several problems exist in producing vaccines directly from pathogenic organisms, as indicated in the last example above. One principle obstacle to using whole bacteria (living or killed) or impure preparations obtained from the bacteria is the presence of many antigenic substances in such preparations that may cause undesirable cross section. For example, protein antigens produced by *S. mutans* have been reported to cross-react with antigens present in human heart muscle and thus, like other pathogenic bacterial proteins, may pose a problem of safety when used in a vaccine for humans.

EP—A—0060129 discloses vaccines for the prevention of gastroenteric disease comprising live, attenuated or killed microorganisms which include plasmids having at least one non-indigenous gene for an adhesion or toxin of a pathogen causing the disease. There is no specific disclosure of lymphoepithelial-homing microorganisms being incapable of long term survival within the vertebrate while retaining the ability to home.

FR—A—2282908 discloses live anti-enteritis vaccines comprising bacterial strains of low pathogenicity modified by the transfer, by classic genetic techniques, of non-recombinant natural plasmids.

DE—A—2530275 discloses live anti-Salmonellosis vaccines in which chromosome fragments coding for a particular antigen of *Salmonella* are transferred, by classic genetic techniques, into a genetically related recipient strain.

EP—A—0048455 (published 31st March 1982) discloses foot and mouth disease virus (FMDV) vaccines produced by recombinant DNA techniques. Among the various expression hosts disclosed are various strains of *E. coli* K12 and χ1776.

EP—A—0068693 (published 5th January 1983) also discloses the production of FMDV vaccine. *E. coli* K12 and χ1776 strains may be used as expression hosts.

3

EP—A—0068719 (published 5th January 1983) discloses the use of *E. coli* χ1776 as a host for the expression of a hepatitis B surface antigen.

Other problems arise in developing a vaccine capable of oral administration, certainly the most desirable form of administration when considered in terms of either widespread use by unskilled administrators in underdeveloped countries or in terms of patient comfort and acceptability. When oral administration is used to stimulate an IgA response, the amount of material that is actually absorbed and capable of stimulating an effective immune response is usually low. The dose of antigen required for oral immunization generally far exceeds that required for systemic induction of immunity. It is assumed that a large portion of the antigen is degraded by enzymes of the gastrointestinal tract and may be eliminated or absorbed in a non-immunogenic form.

Accordingly, this invention enables the provision of a vaccine against bacterial infection that does not have antigenic material associated with it that is capable of causing undesirable antibody-antigen reactions in humans or animals.

The invention also enables the provision of a vaccine that does not have problems of cross-reactivity in a form suitable for oral administration.

Further, the invention enables the provision of a vaccine that will persist in the mucosal system of humans or animals and thereby stimulate the production of IgA in the secretory system.

According to a first aspect of the present invention there is provided a vaccine suitable for the immunization of a vertebrate, which vaccine comprises a pharmaceutically and/or veterinarily acceptable carrier and a non-pathogenic microbe wherein said microbe contains and is capable of expressing a gene, which gene is embodied in a recombinant DNA molecule and derived from a pathogen of a vertebrate, to produce an antigen capable of inducing an immune response in the vertebrate against the pathogen, characterised in that the microbe (a) belongs to a species that homes to a lymphoepithelial structure of a vertebrate and (b) is a viable carrier incapable of reproduction within a vertebrate (while retaining the ability to home to said lymphoepithelial structure) and dies and releases antigens.

The microbe may be a bacterium. Suitable microbes include members of the genera *Escherichia* (such as *E. coli* ᴿDEC-1 and *E. coli* K-12) and *Salmonella* and *Salmonella-Escherichia* hybrids.

The gene may code for a surface antigen or an enzyme, such as glycosyltransferase of the pathogen, which may belong to one of the following species: *Escherichia coli*, *Vibrio cholerae*, *Shigella*, *Pseudomonas aeruginosa*, *Neisseria gonorrhoeae*, *Chlamydia trachomatis* and, particularly, *Streptococcus mutans*.

According to a second aspect of the present invention, there is provided a method of producing a vaccine suitable for immunization of a vertebrate against a pathogen comprising the steps of selecting a gene coding for an antigen of a pathogenic organism, inserting the gene into a carrier microbe in a recombinant DNA molecule, wherein the carrier microbe expresses the gene to produce a gene product capable of inducing antibodies against the pathogenic organism or a metabolic product of the pathogenic organism, and admixing the carrier microbe with a vaccine carrier, characterised in that the carrier microbe (a) belongs to a species that homes to a lymphoepithelial structure of a vertebrate, (b) is a viable carrier incapable of reproduction within a vertebrate (while retaining the ability to home to said lymphoepithelial structure) and dies and releases antigens.

The carrier microbe is preferably non-pathogenic. The gene may be inserted into a plasmid prior to insertion into the carrier microbe.

A more complete appreciation of the invention and many of the attendant advantages thereof will be readily obtained as the invention becomes better understood by reference to the following exemplary detailed description when considered in connection with the accompanying drawings, wherein:

Figure 1 shows an Ouchterlony analysis of reactions between antiserum to purified spaA protein and concentrated culture supernatant fluids from strains of *S. mutans* serotypes a, c, e, f, g and d;

Figure 2 shows an Ouchterlony analysis of antisera to purified *spaA* protein (a *spaA*) and *S. mutans* serotype c antigen I/II (a1/II) with purified *spaA* protein, an extract from *E. coli* χ1274 (pYA726) which produces *spaA* protein and an extract from *E. coli* χ1274 which contains the cloning vector (pACYC184);

Figure 3 shows radiolabelled proteins synthesized in minicells obtained from (1) χ1849 (pBR322), (2) χ1849, (3) χ1849 (pYA601), where the 55k protein is glucosyltransferase and bla=β-lactamase;

Figure 4 shows a physical map of pYA601, with the *S. mutans* DNA insert consisting of two *Hind*III restriction fragments, A and B, of 1360 and 370 bp in length, respectively; and

Figure 5 shows an Ouchterlony analysis of *spaA* protein present in the periplasm (B) and cytoplasm (C) of *E. coli* HB101 (pYA721) where well A contains anti-*spaA* serum.

Microorganisms prepared by the genetic engineering procedures described herein and suitable pathogenic bacteria from which genes may be obtained are exemplified by cultures now on deposit with the American Type Culture collection, 12301 Parklawn Drive, Rockville, Maryland 20852, U.S.A. These cultures are identified as follows:

*Escherichia coli* 1274 (pYA619) (ATCC No. 31984), *Escherichia coli* HB101 (pYA726) (ATCC No. 31985), and *Escherichia coli* 1274 (pYA601) (ATCC No. 31986) strains derived by genetic transfer thereto, and containment therein, of genes from *Streptococcus mutans*; and

*Streptococcus mutans* UAB90 (ATCC No. 31987), *Streptotoccus mutans* UAB50 (ATCC No. 31988), and *Streptococcus mutans* UAB308 (ATCC No. 31989) donor strains.

A sub-culture of each of these strains can be obtained from the collection of the American Culture Collection at the above-given address.

The present inventor has determined that many of the problems associated with previous vaccines against pathogens can be minimized by using non-pathogenic microbes to produce selected antigens from pathogens using recombinant DNA techniques, thus providing these antigens in a form essentially free of contaminating antigens from the pathogenic organism. It is preferred to introduce the vaccine directly to the mucous membranes to stimulate IgA production. Recent studies have shown that a central pathway for the antigenic stimulation of IgA precursor cells exists in gut-associated lymphoid tissues (GALT) and bronchial-associated lymphoid tissues (BALT) and is followed by dissemination of sensitized cels to distant mucosal sites. Available data indicate that the lamina propria of the gut and respiratory tract, mammary and salivary glands, as well as the genitourinary tract, are supplied by sensitized cells from GALT and BALT. For example, lymphocytes are spread throughout the inner layer of the intestine as isolated cells or small cell clusters. Some of the cell clusters develop into distinct organs, known as lymphoepithelial structures. The principal ones in humans are (1) tonsils (in the pharyngeal wall), (2) the appendix (at the junction of the small and large intestine, and (3) Peyer's patches (oblong lymphoid cell clusters found mostly at the terminal portion of the small intestine). For example, Weisz-Carrington *et al*, J. Immunol. *123*, 1705 (1979), have recently shown that oral immunization with ferritin results in the appearance of antibody-producing cells in extra-intestinal lymphoid tissues of mice. Furthermore, in secretory glands, the immunoglobulin class of cells producing anti-ferritin was mostly IgA, while in spleen or peripheral lymph nodes, IgM and IgG ferritin-binding cells were encountered. It is assumed that the major natural pathway for stimulation of the immune system occurs through GALT and BALT sensitization. The schematic representation that follows shows pathways of sensitization of B and T lymphocytes, their emigration from GALT and BALT, homing to distant mucosal tissues and differentiation into IgA-producing plasma cells.

SCHEME I
THE COMMON MUCOSAL IMMUNE SYSTEM
Ingested or Inhalted Antigens

↓

Antigen penetration via specialized mucosal epithelial cells to T
and B lymphocytes in gut or bronchial-associated-lymphoid tissue
(GALT or BALT)

↓

Local Sensitization of T and B cells

↓

Migration of T and B Cells to Mesenteric Lymph Nodes

↓

Thoracic Duct Lymph

↓

Blood Circulation

↓

Homing of B- and T-cells to distant mucosal tissues

| ↓ | ↓ | ↓ | ↓ | ↓ | ↓ |
|---|---|---|---|---|---|
| Lamina Propria of gut and Respiratory Tract | Mammary Glands | Salivary Glands | Lacrymal Glands | Genitourinary Tissues | Others |

↓

Differentiation and clonal expansion to IgA producing
cells (in the presence of antigen)

5

EP 0 080 806 B1

In a preferred embodiment of the invention, carrier bacteria can be used to deliver selected antigens to the GALT, for example to the Peyer's patches of the ileum. Some genera of bacteria, such as Salmonella, are known to home to the Peyer's patches. In a well-studied case with *E. coli* [R]DEC-1, the bacteria specifically attach to, invade and persist in the Peyer's patches of the ileum of rabbits, for which this bacterium is species specific. Other *E. coli* strains specific to the human species are known to colonize Peyer's patches in humans. *S. typhimurium-E. coli* hybrids have also been shown to colonize Peyer's patches in humans. If these carrier bacteria contain and express a recombinant gene from a pathogenic organism, antibodies against the pathogen will be induced. With the advent of recombinant DNA techniques, it now becomes possible to develop totally unique vaccines in which specific antigens are produced, not by the aetiologic agent, but by another host strain of bacteria capable of expressing the gene for that antigen. It is also possible, when antigens might cross-react with an antigen of the mammalian host and thus potentiate the induction of autoimmunity, to use recombinant DNA techniques to alter the gene so that the affecting crossreacting antigenic determinant is not produced. Thus, recombinant DNA techniques can be employed to develop vaccines that do not have any material capable of cross-reacting with mammalian host antigens or capable of eliciting an autoimmune state.

It is apparent that the present invention has wide applicability to the development of effective vaccines against bacterial or viral disease agents where local immunity is important and might be a first line of defense. Some examples are vaccines for the control of enteric diseases caused by enteropathogenic *E. coli* strains, *Vibrio cholerae, Salmonella typhi*, and *Shigella* species. Other vaccines of the invention could be used in the control of persistent *Pseudomonas aeruginosa* infections in patients with cystic fibrosis, in protection against pneumonic plague caused by *Yersinia pestis*, and in prevention of *E. coli* urinary tract infections and of *Neisseria gonorrhoeae, Treponema pallidum* and *Chlamydia trachomatis*-caused venereal diseases as well as *Chlamydia* caused eye infections. Species of *Streptococci* from both group A and group B, such as those species that cause sore throat or heart diseases are additional examples of bacteria within the scope of this invention from which genes could be obtained. Vaccines which contain microbes containing viral genes, such as influenza virus genes, are also encompassed by this invention. Viral genes can also be derived from other viruses, either DNA or RNA viruses.

By vaccine is meant an agent used to stimulate the immune system of a living organism so that protection against future harm is provided. Immunization refers to the process of inducing a continuing high antibody level in an organism, which is directed against a pathogen or antigen to which the organism has been previously exposed. Although the phrase "immune system" can encompass responses of unicellular organisms to the presence of foreign bodies, e.g., interferon production, in this application the phrase is restricted to the anatomical features and mechanisms by which a multi-cellular organism produces antibodies against an antigenic material which invades the cells of the organism or the extra-cellular fluid of the organism. The antibody so produced may belong to any of the immunological classes such as immunoglobulins A, D, E, G, or M. Of particular interest are vaccines which stimulate production of immunoglobulin A (IgA) since this is the principle immunoglobulin produced by the secretory system of warm-blooded animals, although vaccines of the invention are not limited to those which stimulate IgA production. For example, vaccines of the nature described herein are likely to produce a broad range of other immune responses in addition to IgA formation, for example, cellular and humoral immunity. Immune response to antigens is well studied and widely reported. A survey of immunology is given in Davis, Dulbecco *et al,* Microbiology: Immunology and Molecular Genetics, Third Edition, Harper and Row, Hagerstown Md, USA (1980), the entire of which is herein incorporated by reference.

A vertebrate is any member of the subphylum Vertebrata, a primary division of the phylum Chordata that includes the fishes, amphibians, reptiles, birds, and mammals, all of which are characterized by a segmented bony or cartilaginous spinal column. All vertebrates have a functional immune system and respond to antigens by producing antibodies. Thus all vertebrates are capable of responding to vaccines. Although vaccines are most commonly given to mammals, such as humans or dogs (rabies vaccine), vaccines for commercially raised vertebrates of other classes, such as the fishes, if of the nature described herein, are within the scope of the present invention.

One of the essential features of this embodiment of the invention is the use of a non-pathogenic microbe as a carrier of the gene product which is used for stimulating antibody response against a pathogen or allergin. Non-pathogenic does not mean that a microbe of that genus or species can not ever function as a pathogen, but that the particular microbe being used is not a pathogen for the particular animal being treated. The microbe may belong to a genus or even a species that is normally pathogenic but must belong to a strain that is non-pathogenic. By pathogenic is meant capable of causing disease or impairing normal physiological functioning. Microbes include viruses, bacteria, protozoa, and unicellular fungi. It is also possible to use synthetic viruses as carriers comprising viral coat proteins assembled in a test tube with non-viral DNA.

Techniques for transferring genetic material from one organism to another have recently become widely available as the result of rapidly expanding recombinant DNA technology. In this application, genetic material that has been transferred from one organism into a second by recombinant DNA techniques and in such a manner that reproduction of the second organism gives rise to descendents containing the same genetic material is referred to as a recombinant gene. The term gene is being used here in its broadest sense to represent any biological unit of heredity. It is not necessary that the

6

recombinant gene be a complete gene as present in the parent organism, which was capable of producing or regulating the production of a macromolecule, for example, a functioning polypeptide. It is only necessary that the gene be capable of serving as the template used as a guide in the production of an antigenic product. The product may be one that was not found in that exact form in the parent organism. For example, a functional gene coding for a polypeptide comprising 100 amino acid residues may be transferred in part into a carrier microbe so that a peptide comprising only 75, or even 10, amino acid residues is produced by the cellular mechanism of the host cell. However, if this gene product is an antigen that will cause formation of antibodies against a similar antigen present in the parent organism, the gene is considered to be within the scope of the term gene as defined in the present invention. At the other end of the spectrum is a long section of DNA coding for several gene products, one or all of which can be antigenic. Thus a gene as defined and claimed here is any unit of heredity capable of producing an antigen. The gene may be of chromosomal, plasmid, or viral origin.

In order for the gene to be effective in eliciting an immune response, the gene must be expressed as an antigenic product. Expression of a gene means that the information inherent in the structure of the gene (the sequence of DNA bases) is trasnformed into a physical product in the form of a polypeptide, RNA molecule, or other biologic molecule by the biochemical mechanisms of the cell in which the gene is located. These may be the cellular mechanisms of the carrier microbe if the microbe is a bacterium protozoan, or yeast, or the cellular mechanisms of an infected cell if the microbe is a virus. The biological molecule so produced is called the gene product. The term gene product as used here refers to any biological product or products produced as a result of the biochemical reactions that occur under the control of a gene. The gene product will be antigenic, and may be, for example, an RNA molecule, a peptide, or a product produced under the control of an enzyme or other molecule that is the initial product of the gene, i.e., a metabolic product. For example, a gene may first control the synthesis of an RNA molecule which is translated by the action of ribosomes into an enzyme which controls the formation of glycans in the environment external to the original cell in which the gene was found. The RNA molecule, the enzyme, and the glycan are all gene products as the term is used here. Any of these as well as many other types of gene products, such as glycoproteins, will act as antigens if introduced into the immune system of an animal. Protein gene products, including glycoproteins, are preferred gene products for use as antigens in vaccines.

In order for a vaccine to be effective in producing antibodies, the antigenic material must be released in such a way that the antibody-producing mechanism of the vaccinated animal can come into play. Therefore the microbe carrier of the gene product must be introduced into the animal. In order to stimulate a preferred response of the BALT and GALT cells as discussed previously, introduction of the microbe or gene product directly into the gut or bronchus is preferred, although other methods of administering the vaccine, such as intramuscular or subcutaneous injection or intrapenial or vaginal administration, are possible.

Since a carrier microbe is used, once the carrier microbe is present in the animal, the antigen needs to become available to the animal's immune system.

A viable carrier, which is incapable of reproduction and which dies and releases antigen (e.g. cytoplasmic antigens) is therefore used. It is possible to employ a wider range of carrier microbes, for example bacteria that would normally be pathogenic, than when carriers able to reproduce are used. The use of pathogens to deliver antigens from other pathogens to the GALT or BALT would be inappropriate if it were not for the fact that such pathogens can be rendered "avirulent" while retaining ability to invade Peyer's patches. Alteration of *E. coli* strains to preclude their survival in nature is well-known, and has been described in, for example, U.S. Patent 4,190,495, which is herein incorporated by reference. The same deletion (Δ) mutations (e.g., Δ*thyA*) used in the laboratory to create bacteria that require a particular nutrient can be used to preclude long-term survival without preventing penetration of Peyer's patches. The Δ*asd* mutation, which eliminates aspartic acid semialdehyde dehydrogenase and thus imposes a requirement for diaminopimelic acid (DAP), would also result in DAP-less death *in vivo* with release of intracellular and periplasmic proteins. Furthermore, deletion mutations in *S. thyphimurium* that prevent enterochelin synthesis result in strains that are avirulent but which can be used to induce effective immunity against subsequent Salmonella infection. This bacterium is described in Hoiseth and Stocker, Nature *291*, 238 (1981), which is herein incorporated by reference. Such a bacterium provides a convenient source of carrier microbes.

The organism from which the recombinant gene is derived may be any pathogen of the animal being vaccinated or may be an organism that produces an allergen or other antigen capable of inducing an immune response in the animal. Allergens are substances that cause allergic reaction, in this case in the animal which will be vaccinated against them. Many different materials may be allergens, such as animal hair and pollen, and the allergic reaction of individual animals will vary for any particular allergen. It is possible to induce tolerance to an allergen in an animal that normally shows an allergic response. The methods of inducing tolerance are well-known and generally comprise administering the allergen to the animal in increasing dosages. Further discussion of tolerance induction is given in the Davis, Dulbecco *et al.* publication previously cited.

Administration of a live vaccine of the type disclosed above to an animal may be by any known or standard technique. These include oral ingestion, intestinal intubation, or broncho-nasal spraying. All of these methods allow the live vaccine to easily reach the BALT or GALT cells and induce antibody formation and are the preferred methods of administration. Other methods of administration, such as intravenous injection, that allow the carrier microbe to reach the animal's blood stream may be acceptable when the carrier microbe is unable to reproduce. Intravenous injection is also acceptable with other embodiments of the invention using purified antigens, as is described later.

Since preferred methods of administration are oral ingestion and intestinal intubation, preferred carrier microbes are those that belong to species that home preferentially to any of the lymphoepithelial structures of the intestines of the animal being vaccinated. These strains are preferred to be non-pathogenic derivatives of enteropathogenic strains produced by genetic manipulation of enteropathogenic strains. Strains that home to Peyer's patches and thus directly stimulate production of IgA are most preferred. In humans these include strains of *E. coli, Salmonella,* and *Salmonella-E. coli* hybrids that home to the Peyer's patches.

Recombinant DNA techniques are now sufficiently well known and widespread so as to be considered routine. In very general and broad terms, this method consists of transferring the genetic material, or more usually part of the genetic material, of one organism into a second organism so that the transferred genetic material becomes a permanent part of (recombines with) the genetic material of the organisms to which it is transferred. Thus usually consists of first obtaining a small piece of DNA from the parent organism either from a plasmid or a parent chromosome. A plasmid (also called an extrachromosomal element) is a hereditary unit that is physically separate from the chromosome of the cell. The DNA may be of any size and is often obtained by the action of a restriction endonuclease enzyme which acts to split DNA molecules at specific base-pair sites. The dNA pieces may be transferred into a host cell by various means such as transformation (uptake of naked DNA from the external environment, which can be artificially induced by the presence of various chemical agents, such as calcium ions) and transfection (transfer of non-viral DNA by phages or in the presence of phage coat proteins). Other methods such as tranduction are also suitable. Once the parent DNA is in the carrier cell, it may continue to exist as a separate piece (generally true of complete transmitted plasmids) or it may insert into the host cell chromosome and be reproduced with the chromosome during cell division.

Although transferring genetic material is relatively straightforward, predicting which transfers will result in expressed genes is not yet possible. This selection process, however, does not present any difficulty to the present invention. Since the host microbe must express the transferred gene and thereby produce an antigen, a "shotgun" approach works well. Antibodies are first produced against the desired antigen, for example, fragments of cell membranes from pathogenic microbes, by standard techniques. DNA from the organism that is the source of the antigen is cleaved into multiple fragments by endonucleases, and the fragments are inserted randomly into carrier microbes, preferably by means of cloning vectors. The microbes that express antigens from the pathogen can be easily identified by their reaction with antibody against pathogen antigens. Antigen-expressing microbes can be selected and cloned to give the desired recombinant organism. Shotgun cloning is well known and is described in detail in Shinsheimer, Ann. Rev. Biochem. *46*, 415 (1977), which is herein incorporated by reference.

The techniques of gene transfer are not im themselves considered to be part of this invention, and any method capable of producing recombinant organisms comprising genes from pathogenic organisms that are expressed in non-pathogenic microbes will suffice. The techniques of DNA isolation, gene cloning, and related techniques are disclosed in great detail in, for example, *Recombinant DNA*, Methods of Enzymology, Volume 68, Ray Wu, ed., Academic Press (1979), which is herein incorporated by reference.

Vaccines of this invention contain the carrier microbe capable of expressing the pathogen derived gene. The method of administration is preferably varied to fit the type of vaccine being used. Vaccines containing carrier microbes are preferably administered orally, particularly when colonization of Peyer's patches is desired. Dosage will vary depending on the ability of the carrier microbe to express the recombinant gene and thus produce antigen, but will generally be in the 1—10 ml range at concentrations ranging from about $10^2$ to $10^{10}$ microbes per ml. Preferred oral preparations are enteric-coated. Such preparations are resistant to acid and enzymes in the stomach of the inoculated animal while dissolving in the intestines. Various enteric-coating are known in the art, for example as disclosed in U.S. Patent Nos. 3,241,520 and 3,253,944, and are commercially available. A method suitable for preparation of enteric-coated capsules is described in U.S. Patent 4,152,415, which is herein incorporated by reference, and can be easily modified to provide capsules containing the carrier microbes of the present invention. The only required modification is adjustment of the moisture content of the antigen-containing slurry to prevent excessive drying of viable microbes. The amount of drying required is easily determined by routine experimentation.

The pharmaceutical carrier in which the vaccine is suspended or dissolved may be any solvent or solid that is non-toxic to the inoculated animal and compatible with the carrier organism or antigenic gene product. Suitable pharmaceutical carriers include liquid carriers, such as normal saline and other non-toxic salts at or near physiological concentrations, and solid carriers, such as talc or sucrose. Adjuvants, such as Freund's adjuvant, complete or incomplete, may be added to enhance the antigenicity of the gene product if desired. When used for administering via the bronchial tubes, the vaccine is suitably present in the form of an aerosol.

The above disclosure generally describes the present invention. A more complete understanding can be obtained by reference to the following specific example which is provided herein for purposes of illustration only and is not intended to be limiting unless otherwise specified.

Example

DNA from *S. mutans* strains 6715 (serotype g, 45% guanine+cytosine content) and PS14 and GS-5 (serotype c, 35% guanine+cytosine) was cloned into suitable strains of *E. coli* K-12. Shotgun cloning experiments were performed to determine whether *S. mutans* genes were expressed in *E. coli* and, if so, whether they would complement *E. coli* gene defects. The DNA was isolated from the *S. mutans* strains UAB50 (ATCC 31987), UAB90 (ATCC 31988), and UAB308 (ATCC 31989) by treating the *S. mutans* cells with the enzyme mutanolysin and then lysing the bacteria with the detergent sodium dodecyl sulphate. The DNA was recovered by ethanol precipitation, restricted with various restriction endonucleases such as *EcoRI*, *HindIII*, *BamHI*, and *PstI* and used to anneal to pBR322 or pACYC184 vectors cut with the homologous enzyme. Recombinant molecules were formed by the addition of polynucleotide joining enzyme (or DNA ligase) and suitable strains of *E. coli* K-12 such as HB101, χ1274, and χ1849 were transformed by the calcium chloride cold shock method. In other experiments, recombinant molecules were formed by ligating DNa to the cosmid vector pJC74, packaging the recombinant molecules by *in vitro* packaging methodology with components to introduce the recombinant cosmid DNA into suitable strains of *E. coli* K-12 such as HB101 lysogenic for the thermo-inducible lambda phosphage λ *cl857*. Transformant or transfectant clones were selected for resistance to an antibiotic for which the cloning vector carried the appropriate drug resistance gene. Tests using a variety of multiple mutant *E. coli* strains indicated that about 40% of the tested *E. coli* gene defects for purine, pyrimidine and amino acid biosynthesis and carbohydrate use could be complemented by *S. mutans* genetic information. The presence of *S. mutans* DNA was verified using Southern blotting analysis. *E. coli* deletion mutants lacking a given function would sometimes grow as rapidly with *S. mutans* genetic information cloned on the multicopy plasmids pBR322 and pACYC184 (available commercially from Bethesda Research Laboratory, Rockville, Md. USA) as they would if provided with the optimal amount of the required supplement. In general, most *S. mutans* genes were expressed constitutively and were not subject to repression by end products or inducible by appropriate substrates. One exception was a cluster of four or five *S. mutans* genes for galactose utilization. These genes were coordinately regulated and moderately inducible in *E. coli* in the same manner as they were in *S. mutans*. This suggested some type of autogenous regulation, an hypothesis currently being tested. Gene products of *S. mutans* that are necessary for the transport and phosphorylation of sugars and that probably associate with the cytoplasmic membrane of *S. mutans* function in *E. coli* in much the same way. *S. mutans* gene products that are normally on the cell surface of *S. mutans* or are excreted into the growth medium were transported across the *E. coli* cytoplasmic membrane and ended up in the periplasmic space. *E. coli perA* mutants that are defective in transport of various periplasmic proteins from the cytoplasm into the periplasm were still able to transport certain *S. mutans* cell surface gene products into the periplasmic space in *E. coli*.

A goal in cloning *S. mutans* genes in *E. coli* was to identify those genes that might contribute to the ability of *S. mutans* to colonize the tooth surface. Glucosyltransferase cleaves sucrose to yield fructose while polymerizing guclose into glucans. *S. mutans* synthesizes both water-soluble glucans which have predominantly α 1→6 linkages and water-insoluble glucans which are branched and have α 1→3 linkages in addition to the α 1→6 linkages. A gene for glucosyltransferase activity from both *S. mutans* PS14 and GS-5 was cloned into *E. coli*. This gene in pYA601 (from PS14) and pYA619 (from GS-5) is contained on a 1730 base pair DNA fragment which encodes an enzyme that synthesizes water-soluble glucans. This glucosyltransferase, which has a 55,000 molecular weight, is transported into the *E. coli* periplasm without modification. There it is able to cleave sucrose (which can get into *E. coli*'s periplasmic space) to liberate fructose which can be used for growth and to synthesize glucans. As previously mentioned, immunization with purified glucosyltransferase conferred protective immunity against *S. mutans* induced dental caries.

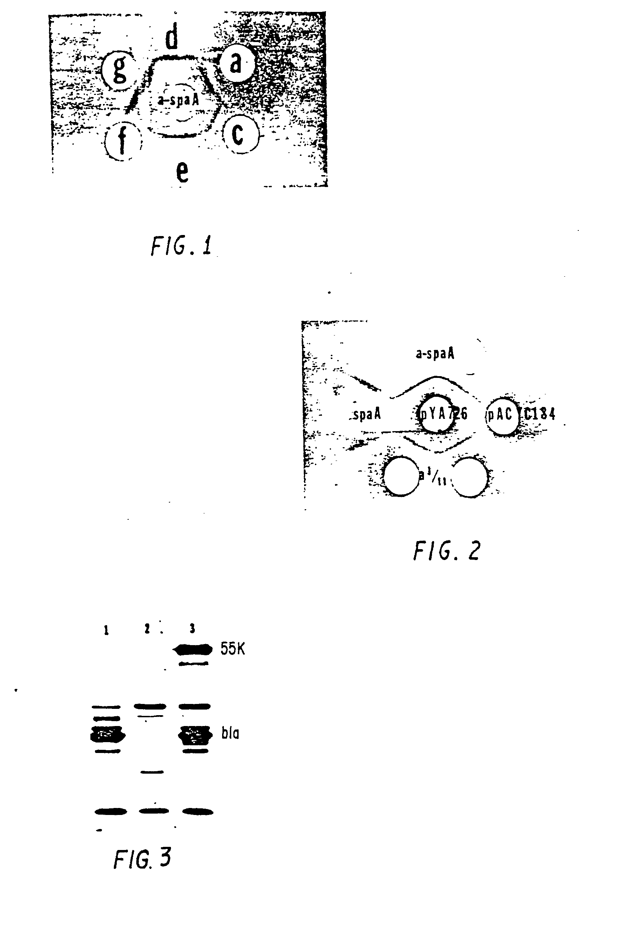

In order to successfully clone various *S. mutans* genes for cell-surface associated proteins that might not have enzymatic activity, an immunological screening procedure was used. In this approach, antibodies were raised against ammonium sulphate precipitated extracellular proteins of *S. mutans* 6715. The pJC74 cosmid cloning system was used and the recombinant molecules were introduced into an *E. coli* strain lysogenic for the λ*cl857* thermosensitive prophage. This cloning system is described in Collins et al, PNAS, *75*, 4242 (1978), which is herein incorporated by reference. Induction of lysogenic cells in colonies grown at 30°C by shifting the temperature to 37°C caused cell lysis to release any antigens, even if they were localized to the cytoplasm rather than being transported to the exterior surface of *E. coli*. Using this method and the formation of precipitin bands around the colonies containing antigens cross-reacting with the antibodies against *S. mutans* extracellular proteins, it was possible to identify numerous clones which expressed *S. mutans* cell surface associated proteins antigens. These clones were given the identifications pYA701 through pYA721. One clone (pYA721) was of particular interest. This clone specifies a 210 k protein (the *spaA* protein) which was originally encoded by a 5.5 Mdal fragment of *S. mutans* DNA. This particular protein antigen cross-reacts with protein antigens in all of the serotypes of *S. mutans* except for serotype b which is a group of *S. mutans* (correctly known as *S. rattus*) that principally colonizes rodents and not humans. An Ouchterlony analysis of this cross-section is shown in Fig. 1.

It should be noted that this 210 k protein from serotype g *S. mutans* is immunologically related to the antigen I/II (Fig. 2) of Russel who found that injection of this protein purified from the serotype c *S. mutans* strain Ingbritt into Rhesus monkeys was protective against *S. mutans* induced dental caries (Russel *et al.,* Immunol, *40*, 97 (1980)).

Immunization of the submandibular region of gnotobiotic rats were killed *S. mutans* cells induces salivary agglutinins against *S. mutans* which are in the IgA class. Such immunization was protective against subsequent challenge with virulent *S. mutans* strains in that mean caries scores were significantly lower in the immunized rats than in the control group that had not been immunized. Subsequent studies demonstrated that ingestion of killed *S. mutans* cells would likewise stimulate salivary secretory IgA against *S. mutans* and also confer protective immunity when rats were subsequently challenged with virulent *S. mutans* strains. These results have been confirmed in human studies in which it was shown that oral ingestion of killed *S. mutans* resulted in production of sIgA in tears and saliva. These antibodies were specific against the *S. mutans* vaccine strain. These studies also indicated a negligible increase in serum agglutinins and gave no evidence of stimulation of any human heart reactive antibodies.

The two *S. mutans* gene products expressed in *E. coli* that have been most intensely studied have been the *spaA* protein and glucosyltransferase (GTF).

Glucosyltransferase can be assayed by its ability to hydrolyze sucrose to liberate reducing sugars. The Somogyi reagent is used and the reaction is followed at 520 nm. This assay can be used on extracts and on toluenized cells. Enzyme activity in non-denaturing polyacrylamide gels can be measured by soaking the gels in 0.1 M sucrose (in 0.2 M phospahte buffer, pH 7.0) for 1 to 2 h followed by treating with triphenyl tetrazolium chloride in alkali in the dark to identify the band(s) containing reducing sugar generating activity. It is also possible to assay the GTF for synthesis of glucan using uniformly labelled radioactive sucrose but this is more costly and time consuming. Monospecific rabbit antibody against GTF produced by E. coli recombinants and monoclonal antibodies can be used for radioimmunoassay (RIA) and the antibody tagged with fluorescein or rhodamine isothiocyanate can be used to identify bacterial cells that have GTF on their cell surface.

In terms of purification, GTF is a 55 k protein (Fig. 3) which is soluble in 33% ammonium sulfate. Many *E. coli* proteins are thus removed from the cell extract by ammonium sulfate precipitation. This is followed by a combination of chromatography on diethylaminoethyl (DEAE) cellulose (DE52; Whatman Ltd.), Ultrogel® AcA54 and Ultrogel® AcA44 (LKB) with concentration of pools by ultrafiltration with Amicon membranes to result in a homogeneous GTF preparation. Protein contaminants are less than a few percent since other protein bands are undetectable by either radioactive labelling and radioautography or by Comassie blue straining of SDS polyacrylamide gels. Using this method about 50% GTF recovery can be achieved and some 10 to 15 mg of GTF from a one litre culture of *E. coli* cells harbouring the pYA601 plasmid have been obtained.

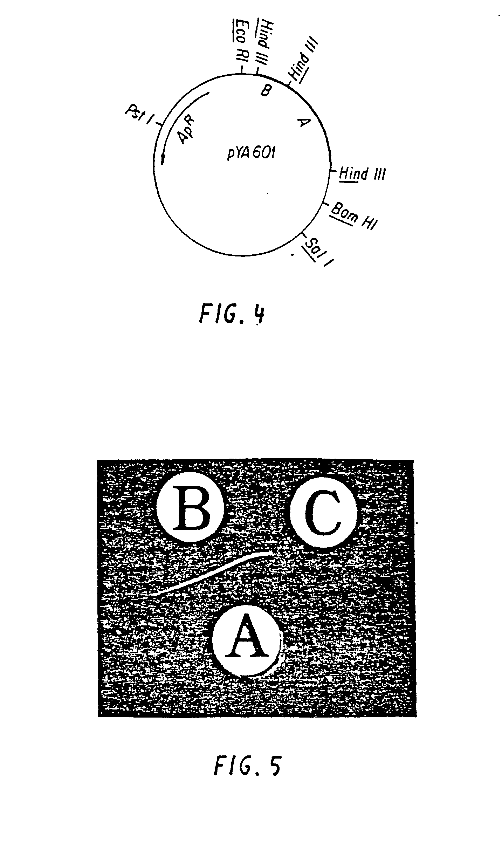

GTF is synthesized constitutively in *E. coli* χ1849. *E. coli* cells growing on glucose or raffinose with isopropyl - thio - β - galactopyranoside (IPTG) produce about 50% as many glucosyltransferase molecules as β-lactamase molecules which are also encoded on the pBR322 cloning vector (Fig. 3). The gene for glucosyltransferase spans two *Hind*III fragments of 1360 and 370 bp (Fig. 4). The larger fragment contains the RNA polymerase binding site. (Both fragments have also been cloned within a *Bam*HI fragment in pYA619). If one subtracts 100 bp for promoter and terminator control sequences, then the two *Hind*III fragments would specify a protein of only 55 to 58 k in molecular weight.

Because of its size, the *SpaA* protein can be purified in several steps. When the protein is purified from *S. mutans*, the cell-free culture supernatant fluid is concentrated to 5% of its original volume by ultrafiltration, sucrose is added to a concentration of 10% (w/v) and the fraction is incubated at 37°C for 2 hrs. All glucosyltransferase enzymes along with other proteins that bind to dextrans or have affinity for sucrose form complexes that are removed by low speed centrifugation. The supernatant fluid is then run successively on Ultrogel® AcA44 and Ultrogel® AcA34. Finally, a homogeneous protein is obtained after DEAE cellulose chromatography. In *E. coli*, the 218 k *spaA* protein can be purified from the periplasmic fluid released by cold osmotic shock. Since there are few proteins of this size in the *E. coli* periplasm, one can obtain homogeneous preparations of this protein by ion exchange chromatography on DEAE cellulose followed by gel filtration chromatography on Ultrogel® AcA34.

The *spaA* protein is assayed immunologically. Monospecific rabbit antibodies against the *spaA* protein made by both *S. mutans* and *E. coli* have been made. These antibodies have been used to measure the amount of *spaA* protein in the *E. coli* periplasm (over 80%) vs. the *E. coli* cytoplasm using both agar double diffsion (Fig. 5) and rocket immunoelectrophoresis. The antibody has also been conjugated against the *spaA* protein with rhodamine isothiocyanate to screen Streptococcal cells from the presence of *spaA* protein on their cell surface. This fluorescent antibody can be used to identify *E. coli* strains expressing the *spaA* protein on their cell surface.

The pharmaceutical carrier in which the vaccine is suspended or dissolved may be any solvent or solid that is non-toxic to the inoculated animal and compatible with the carrier organism or antigenic gene product. Suitable pharmaceutical carriers include liquid carriers, such as normal saline and other non-toxic salts at or near physiological concentrations, and solid carriers, such as talc or sucrose. Adjuvants, such as Freund's adjuvant, complete or incomplete, may be added to enhance the antigenicity of the gene product if desired. When used for administering via the bronchial tubes, the vaccine is suitably present in the form of an aerosol.

The invention now being fully described, it will be apparent to one of ordinary skill in the art that many changes and modifications can be made thereto without departing from the spirit or scope of the invention as set forth herein.

**Claims for the Contracting States: BE, CH, DE, FR, GB, IT, LI, LU, NL, SE**

1. A vaccine suitable for the immunization of a vertebrate, which vaccine comprise a pharmaceutically and/or verterinarily acceptable carrier and a non-pathogenic microbe wherein said microbe contains and is capable of expressing a gene, which gene is embodied in a recombinant DNA molecule and derived from a pathogen of a vertebrate, to produce an antigen capable of inducing an immune response in the vertebrate against the pathogen, characterised in that the microbe (a) belongs to a species that homes to a lymphoepithelial structure of a vertebrate and (b) is a viable carrier incapable of reproduction within a vertebrate (while retaining the ability to home to said lymphoepithelial structure) and dies and releases antigens.

2. A vaccine as claimed in Claim 1, wherein the microbe is a bacterium.

3. A vaccine as claimed in Claim 1 or 2, wherein the gene codes for a surface antigen of the pathogen.

4. A vaccine as claimed in Claim 1, 2 or 3, wherein the gene codes for an enzyme of the pathogen.

5. A vaccine as claimed in any one of Claims 1 to 4, wherein the pathogen is a bacterium.

6. A vaccine as claimed in Claim 1 or 2, wherein the microbe is a bacterium that belongs to the genus *Salmonella* or *Escherichia* or is *Salmonella-Escherichia* hybrid.

7. A vaccine as claimed in Claim 5, wherein the bacterium belongs to a species selected from the group consisting of *Escherichia coli, Vibrio Cholerae, Shigella, Pseudomonas aeruginosa, Neisseria gonorrhoeae, Chlamydia trachomatis,* and. *Streptococcus mutans.*

8. A vaccine as claimed in Claim 6, wherein the bacterium is an *E. coli* [R]DEC-1 strain or an *E. coli* K-12 strain which is a viable carrier incapable of reproduction within a vertebrate (while retaining the ability to home to the lymphoepithelial structure) and dies and releases antigens.

9. A vaccine as claimed in Claim 1 or 2, wherein the pathogen is *Streptococcus mutans* and the microbe is an *Escherichia coli* or *Salmonella* bacterium, or is a *Salmonella-Escherichia* hybrid.

10. A vaccine as claimed in any one of Claims 1 to 9, wherein the gene codes for a surface antigen or a glycosyltransferase enzyme.

11. A method of producing a vaccine suitable for immunization of a vertebrate against a pathogen comprising the steps of selecting a gene coding for an antigen of a pathogenic organism, inserting the gene into a carrier microbe in a recombinant DNA molecule, wherein the carrier microbe, expresses the gene to produce a gene product capable of inducing antibodies against the pathogenic organism or a metabolic product of the pathogenic organism, and admixing the carrier microbe with a vaccine carrier, characterised in that the vaccine carrier (a) belongs to a species that homes to a lymphoepithelial structure of a vertebrate, (b) is incapable of reproduction within a vertebrate (while retaining the ability to home to said lymphoepithelial structure) and dies and releases antigens.

12. A method as claimed in Claim 11, wherein the carrier microbe is a bacterium.

13. A method as claimed in Claim 11 or 12, wherein the gene codes for a surface antigen or enzyme of the pathogen.

14. A method as claimed in any one of Claims 11 to 13, wherein the bacterium belongs to the genus *Salmonella* or *Escherichia* or is a *Salmonella-Escherichia* hybrid.

15. The method of any one of Claims 11 to 14, wherein the pathogenic organism is *Streptococcus mutans.*

16. A method as claimed in any one of Claims 11 to 15, wherein the carrier microbe is non-pathogenic.

17. A method as claimed in Claim 16, wherein the vaccine comprises said gene product and the carrier microbe.

18. A method as claimed in any one of Claims 11 to 17, wherein the gene is inserted into a plasmid prior to insertion into the carrier microbe.

**Claims for the Contracting State: AT**

1. A method of producing a vaccine suitable for immunization of a vertebrate against a pathogen comprising the steps of selecting a gene coding for an antigen of a pathogenic organism, inserting the gene into a carrier microbe in a recombinant DNA molecule, wherein the carrier microbe expresses the gene to produce a gene product capable of inducing antibodies against the pathogenic organism or a metabolic product of the pathogenic organism, and admixing the carrier microbe with a vaccine carrier, characterised in that the carrier microbe (a) belongs to a species that homes to a lymphoepithelial structure of a vertebrate, (b) is a viable carrier incapable of reproduction within a vertebrate (while retaining the ability to home to said lymphoepithelial structure) and dies and releases antigens.

2. A method as claimed in Claim 1, wherein the carrier microbe is a bacterium.

3. A method as claimed in Claim 1 or 2, wherein the gene codes for a surface antigen or enzyme of the pathogen.

4. A method as claimed in any one of Claims 1 to 3, wherein the bacterium belongs to the genus *Salmonella* or *Escherichia* or is a *Salmonella-Escherichia* hybrid.

5. A method as claimed in any one of Claims 1 to 4, wherein the pathogenic organism is *Streptococcus mutans*.

6. A method as claimed in any one of Claims 1 to 5, wherein the carrier microbe is non-pathogenic.

7. A method as claimed in Claim 6, wherein the vaccine comprises said gene product and the carrier microbe.

8. A method as claimed in any one of Claims 1 to 7, wherein the gene is inserted into a plasmid prior to insertion into the carrier microbe.

**Patentansprüche für die Vertragsstaaten: BE, CH, DE, FR, GB, IT, LI, LU, NL, SE**

1. Impfstoff für die Immunisierung eines Wirbeltieres, welcher Impfstoff einen pharmazeutisch und/oder veterinär verträglichen Träger und einen nicht-pathogenen Mikroorganismus enthält worin der Mikroorganismus ein Gen enthät und fähig ist dieses Gen anzuregen, welches Gen in ein rekombinantes DNA-Molekül eingefügt und von einem Erreger eines Wirbeltieres abgeleitet ist, um ein Antigen zu bilden welches fähig ist eine Immun-Antwort in dem Wirbeltier gegen den Erreger zu induzieren, dadurch gekennzeichnet, daß der Mikroorganismus (a) zu einer Spezies gehört welche sich in einer lymphoepithelischen Struktur eines Wirbeltieres festsetzt und (b) ein lebensfähiger Träger ist, welcher unfähig ist sich in dem Wirbeltier zu reproduzieren (während die Fähigkeit sich in dieser lymphoepithelischen Struktur festzusetzen beibehalten bleibt) und stirbt und Antigene freisetzt.

2. Impfstoff nach Anspruch 1, worin der Mikroorganismus ein Bakterium ist.

3. Impfstoff nach Anspruch 1 oder 2, worin des Gen für ein Oberflächenantigen des Erregers kodiert.

4. Impfstoff nach Anspruch 1, 2 oder 3, worin das Gen für ein Enzym des Erregers kodiert.

5. Impfstoff nach einem der Ansprüche 1 bis 4, worin der Erreger ein Bakterium ist.

6. Impfstoff nach Anspruch 1 oder 2, worin der Mikroorganismus ein Bakterium ist, welches der Gattung Salmonella oder Escherichia angehört oder das Salmonella-Escherichia Hybrid ist.

7. Impfstoff nach Anspruch 5, worin das Bakterium einer Spezies gewählt aus der Gruppe bestehend aus Escherichia coli, Vibrio Cholerae, Shigella, Pseudomonas aeruginosa, Neisseria gonorrhoeae, Chlamydia trachomatis und Streptococcus mutans angehört.

8. Impfstoff nach Anspruch 6, worin das Bakterium ein E. coli $^R$DEC-1 Stamm oder ein E. coli K-12 Stamm ist, welcher ein lebensfähiger, zur Reproduktion in einem Wirbeltier unfähiger Träger ist (während die Fähigkeit sich in dieser lymphoepithelischen Struktur festzusetzen beibehalten bleibt) und stirbt und Antigene freisetzt.

9. Impfstoff nach Anspruch 1 oder 2, worin der Erreger Streptococcus mutans ist und der Mikroorganismus ein Escherichia coli oder Salmonella Bakterium, oder ein Salmonella-Escherichia Hybrid ist.

10. Impfstoff nach einem der Ansprüche 1 bis 9, worin das Gen für ein Oberflächenantigen oder ein Glycosyltransferase-Enzym kodiert.

11. Verfahren zur Herstellung eines Impfstoffes für die Immunisierung eines Wirbeltieres gegen einen Erreger, welches die Schritte der Auswahl eines Gens, welches für ein Antigen des Erregerorganismus kodiert, des Einsetzens des Gens in einer Träger-Mikroorganismus in einem rekombinanten DNA Molekül, worin der Träger-Mikroorganismus das Gen anregt ein Genprodukt zu bilden welches fähig ist Antikörper gegen den Erregerorganismus oder ein metabolisches Produkt des Erregerorganismus zu induzieren, und das Vermischen des Träger-Mikroorganismus mit einem Impfstoffträger umfaßt dadurch gekennzeichnet, daß der Impfstoffträger (a) zu einer Spezies gehört welche sich in einer lymphoepithelischen Struktur des Wirbeltieres festsetzt (b), zur Reproduktion in den Wirbeltier unfähig ist (während die Fähigkeit sich in dieser lymphoepithelischen Struktur festzusetzen beibehalten bleibt) und stirbt und Antigene freisetzt.

12. Verfahren nach Anspruch 11, worin der Träger-Mikroorganismus ein Bakterium ist.

13. Verfahren nach Anspruch 11 oder 12, worin das Gen für ein Oberflächenantigen oder Enzym des Erregers kodiert.

14. Verfahren nach einem der Ansprüche 11 bis 13, worin das Bakterium der Gattung Salmonella oder Escherichia angehört oder ein Salmonella-Escherichia Hybrid ist.

15. Verfahren nach einem der Ansprüche 11 bis 14, worin der Erregerorganismus Streptococcus mutans ist.

16. Verfahren nach einem der Ansprüche 11 bis 15, worin der Träger-Mikroorganismus nicht-pathogen ist.

17. Verfahren nach Anspruch 16, worin der Impfstoff das Genprodukt und den Träger-Mikroorganismus enthält.

18. Verfahren nach einem der Ansprüche 11 bis 17, worin das Gen vor der Insertion in den Träger-Mikroorganismus in ein Plasmid eingeführt wird.

**Patentansprüche für den Vertragsstaat: AT**

1. Verfahren zur Herstellung eines Impfstoffes für die Immunisierung eines Wirbeltieres gegen einen Erreger, welches die Schitte des Auswahl eines Gens, welches für ein Antigen des Erregerorganismus kodiert, des Einsetzens des Gens in einen Träger-Mikroorganismus in einem rekombinanten DNA Molekül, worin der Träger-Mikroorganismus das Gen anregt ein Genprodukt zu bilden welches fähig ist Antikörper gegen den Erregerorganismus oder ein metabolisches Produkt das Erregerorganismus zu indizieren, und das Vermischen des Träger-Mikroorganismus mit einem Impfstoffträger umfaßt dadurch gekennzeichnet, daß der Impfstoffträger (a) zu einer Spezies gehört welches sich in einer lymphoepithelischen Struktur des Wirbeltieres festsetzt (b) ein lebensfähiger Träger ist, welcher unfähig ist sich in dem Wirbeltier zu reproduzieren (während die Fähigkeit sich in dieser lymphoepithelischen Struktur festzusetzen beibehalten bleibt) und stirbt und Antigene freisetzt.

2. Verfahren nach Anspruch 1 worin der Träger-Mikroorganismus ein Bakterium ist.

3. Verfahren nach Anspruch 1 oder 2, worin das Gen für ein Oberflächenantigen oder Enzym des Erregers kodiert.

4. Verfahren nach einem der Ansprüche 1 bis 3, worin das Bakterium der Gattung Salmonella oder Escherichia angehört oder ein Salmonella-Escherichia Hybrid ist.

5. Verfahren nach einem der Ansprüche 1 bis 4, worin der Erregerorganismus Streptococcus mutans ist.

6. Verfahren nach einer der Ansprüche 1 bis 5, worin der Träger-Mikroorganismus nicht-pathogen ist.

7. Verfahren nach Anspruch 6, worin der Impfstoff das Genprodukt und den Träger-Mikroorganismus enthält.

8. Verfahren nach einem der Ansprüche 1 bis 7, worin das Gen vor der Insertion in den Träger-Mikroorganismus in ein Plasmid eingeführt wird.

**Revendications pour les Etats Contractants: BE, CH, DE, FR, GB, IT, LI, LU, NL, SE**

1. Vaccin approprié pour l'immunisation d'un vertébré, qui comprend un support acceptable du point de vue pharmaceutique et/ou vétérinaire et un microbe non pathogène et dans lequel le microbe contient un gène et est capable de l'exprimer, ce gène étant incorporé dans une molécule d'ADN recombinant et étant dérivé d'un agent pathogène d'un vertébré, en vue de produire un antigène capable d'induire dans le vertébré une réponse immunitaire vis-à-vis de l'agent pathogène, caractérisé en ce que (a) le microbe appartient à une espèce qui retourne vers une structure lymphoépithéliale d'un vertébré et en ce que (b) il est un support viable incapable de se reproduire à l'intérieur d'un vertébré (tout en retenant la capacité de retourner vers ladite structure lymphoépithéliale) et en ce qu'il meurt et libère des antigènes.

2. Vaccin suivant la revendication 1, caractérisé en ce que le microbe est une bactérie.

3. Vaccin suivant l'une des revendications 1 et 2, caractérisé en ce que le gène est codant pour un antigène de surface de l'agent pathogène.

4. Vaccin suivant l'une des revendications 1 à 3, caractérisé en ce que le gène est codant pour une enzyme de l'agent pathogène.

5. Vaccin suivant l'une quelconque des revendications 1 à 4, caractérisé en ce que l'agent pathogène est une bactérie.

6. Vaccin suivant l'une des revendications 1 et 2, caractérisé en ce que le microbe est une bactérie qui appartient au genre *Salmonella* ou *Escherichia* ou est un hybride de *Salmonella-Escherichia*.

7. Vaccin suivant la revendication 5, caractérisé en ce que le bactérie appartient à une espèce choisie parmi le groupe comprenant *Escherichia coli, Vibrio cholerae, Shigella, Pseudomonas aeruginosa, Neisseria gonorrhoeae, Chlamydia trachomatis,* et *Streptococcus mutans*.

8. Vaccin suivant la revendication 6, caractérisé en ce que le bactérie est une souche d'*E. coli* [R]DEC-1 ou une souche d'*E. coli* K-12 qui est un support viable incapable de se reproduire à l'intérieur d'un vertébré (tout en retenant la capacité de retourner vers la structure lymphoépithéliale) et qui meurt et libère des antigènes.

9. Vaccin suivant l'une des revendications 1 et 2, caractérisé en ce que l'agent pathogène est *Streptococcus mutans* et en ce que le microbe est une bactérie *Escherichia coli* ou *Salmonella* ou un hybride de *Salmonella-Escherichia*.

10. Vaccin suivant l'une quelconque des revendications 1 à 9, caractérisé en ce que le gène est codant pour un antigène de surface ou une enzyme de glycosyltransférase.

11. Procédé de préparation d'un vaccin approprié pour l'immunisation d'un vertébré vis-à-vis d'un agent pathogène, comprenant la sélection d'un gène codant pour un antigène d'un organisme pathogène, l'introduction du gène dans un microbe porteur dans une molécule d'ADN recombinant, où le microbe porteur exprime le gène, pour produire un produit de gène capable d'induire des anticorps vis-à-vis de l'organisme pathogène ou un produit métabolique de l'organisme pathogène, et le mélange du microbe porteur et d'un support de vaccin, caractérisé en ce que (a) le microbe porteur appartient à une espèce qui retourne vers une structure lymphoépithéliale d'un vertébré et en ce que (b) il est incapable de se reproduire à l'intérieur d'un vertébré (tout en retenant la capacité de retourner vers ladite structure lymphoépithéliale) et en ce qu'il meurt et libère des antigènes.

12. Procédé suivant la revendication 11, caractérisé en ce que le microbe porteur est une bactérie.

13. Procédé suivant l'une des revendications 11 et 12, caractérisé en ce que le gène est codant pour un antigène de surface ou une enzyme de l'agent pathogène.

13

14. Procédé suivant l'une quelconque des revendications 11 à 13, caractérisé en ce que la bactérie appartient au genre *Salmonella* ou *Escherichia* ou est un hybride de *Salmonella-Escherichia*.

15. Procédé suivant l'une quelconque des revendications 11 à 14, caractérisé en ce que l'organisme pathogène est *Streptococcus mutans*.

16. Procédé suivant l'une quelconque des revendications 11 à 15, caractérisé en ce que le microbe porteur est non pathogène.

17. Procédé suivant la revendication 16, caractérisé en ce que le vaccin comprend ledit produit de gène et le microbe porteur.

18. Procédé suivant l'une quelconque des revendications 11 à 17, caractérisé en ce que le gène est introduit dans un plasmide avant l'introduction dans le microbe porteur.

**Revendications pour l'Etat Contractant: AT**

1. Procédé de préparation d'un vaccin approprié pour l'immunisation d'un vertébré vis-à-vis d'un agent pathogène, comprenant la sélection d'un gène codant pour un antigène d'un organisme pathogène, l'introduction du gène dans un microbe porteur dans une molécule d'ADN recombinant, où le microbe porteur exprime le gène, pour produire un produit de gène capable d'induire des anticorps vis-à-vis de l'organisme pathogène ou un produit métabolique de l'organisme pathogène, et le mélange du microbe porteur et d'un support de vaccin, caractérisé en ce que (a) le microbe porteur appartient à une espèce qui retourne vers une structure lymphoépithéliale d'un vertébré et en ce que (b) il est incapable de se reproduire à l'intérieur d'un vertébré (tout en retenant la capacité de retourner vers ladite structure lymphoépithéliale) et en ce qu'il meurt et libère des antigènes.

2. Procédé suivant la revendication 1, caractérisé en ce que le microbe porteur est une bactérie.

3. Procédé suivant l'une des revendications 1 et 2, caractérisé en ce que le gène est codant pour un antigène de surface ou une enzyme de l'agent pathogène.

4. Procédé suivant l'une quelconque des revendications 1 à 3, caractérisé en ce que la bactérie appartient au genre *Salmonella* ou *Escherichia* ou est un hybride de *Salmonella-Escherichia*.

5. Procédé suivant l'une quelconque des revendications 1 à 4, caractérisé en ce que l'organisme pathogène est *Streptococcus mutans*.

6. Procédé suivant l'une quelconque des revendications 1 à 5, caractérisé en ce que le microbe porteur est non pathogène.

7. Procédé suivant la revendication 6, caractérisé en ce que le vaccin comprend ledit produit de gène et le microbe porteur.

8. Procédé suivant l'une quelconque des revendications 1 à 7, caractérisé en ce que le gène est introduit dans un plasmide avant l'introduction dans le microbe porteur.

FIG. 1

FIG. 2

FIG. 3

FIG. 4

FIG. 5